# EUROPEAN PATENT SPECIFICATION

(11) **EP 2 630 497 B1**
(45) Date of publication and mention of the grant of the patent: **03.08.2016**
(21) Application number: 11796771.1
(22) Date of filing: 19.10.2011
(51) Int. Cl.: G01N 33/574

(54) **QUANTITATION OF URINARY TISSUE FACTOR FOR THE DIAGNOSIS AND SCREENING OF CANCER**
BESTIMMUNG VON URIN GEWEBEFAKTOR ZUR KREBS-DIAGNOSE UND -SCREENING
ANALYSE QUANTITATIVE DE FACTEUR DE TISSU URINAIRE POUR LE DIAGNOSTIC ET LE DÉPISTAGE D'UN CANCER

(30) Priority: 19.10.2010 GB 201017615
(43) Date of publication of application: 28.08.2013
(73) Proprietor: C-Term Diagnostics Limited, Sutton Coldfield B74 2UG (GB)
(72) Inventor: HERD, Thomas Mole, Hampshire SO50 8QA (GB); GREENFIELD, Robert, Irvington, New York 10533 (US)
(74) Representative: Maschio, Antonio
(86) International application number: PCT/GB2011/052026
(87) International publication number: WO 2012/052762

(56) References cited:
- US-A1- 2003 119 075
- CARSON; YODER: BLOOD COAGUL FIBRINOLYSIS, vol. 3, 1992, pages 779-787, XP9156520,
- ADAMSON A S ET AL: "Urinary tissue factor levels in prostatic carcinoma: A potential marker of metastatic spread?", BRITISH JOURNAL OF UROLOGY, BLACKWELL PUBLISHING LTD, GB, vol. 71, no. 5, 1 January 1993 (1993-01-01), pages 587-592, XP009156472, ISSN: 0007-1331
- FAREED J ET AL: "Tissue factor antigen levels in various biological fluids", BLOOD COAGULATION & FIBRINOLYSIS, RAPID COMMUNICATIONS, OXFORD, OXFORD, GB, vol. 6, no. Suppl.1, 1 June 1995 (1995-06-01), pages S32-S36, XP009156474, ISSN: 0957-5235
- LWALEED B A: "Increased urinary tissue factor levels in patients with advanced bladder cancer", UROONCOLOGY, TAYLOR & FRANCIS HEALTH SCIENCES, ABINGDON, GB, vol. 2, no. 2, 1 January 2002 (2002-01-01) , pages 103-104, XP009156468, ISSN: 1561-0950, DOI: 10.1080/1561095021000003133

## Description

### Tissue Factor

A series of coordinated reactions takes place in the body whenever blood clots. The major physiological Initiator of these reactions is a membrane-bound glycoprotein known as Tissue Factor (TF), which is normally separated from the blood stream by the vascular endothelium. Bleeding, caused by injury or tissue damage, activates a complex enzyme cascade as TF becomes exposed to the bloodstream.

However, In disease states, leucocytes or the vascular endothelium may abnormally express to cause intravascular coagulation. The blood coagulation cascade Is also relevant to diseases such as haemophilia in which sufferers are deficient in blood proteins necessary for normal clotting, and is linked to vascular diseases such as heart attacks and strokes In which clotting may lead to occlusion of blood vessels.

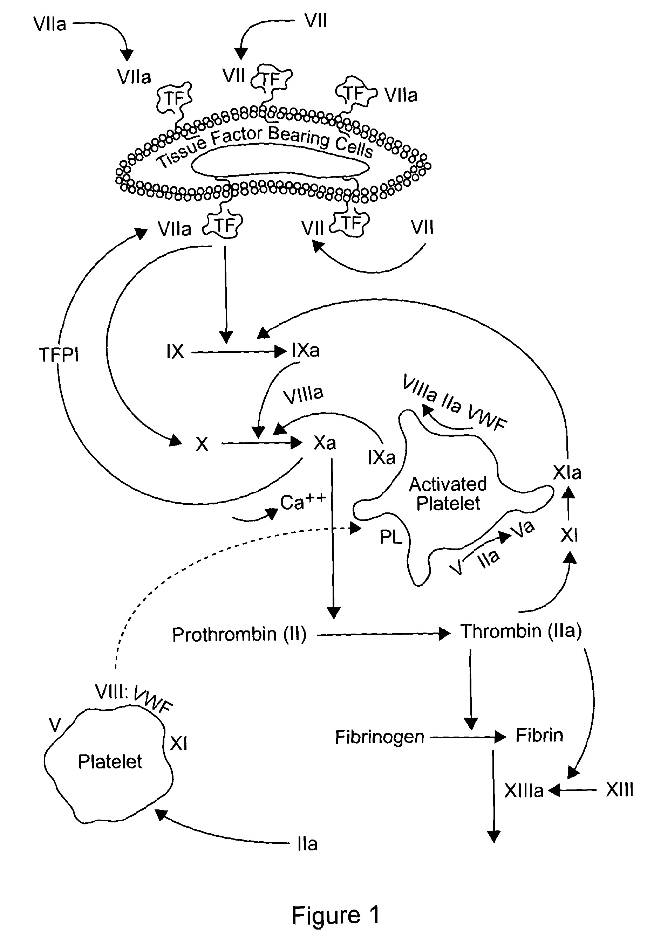

Coagulation Is also activated In inflammatory Illnesses and cancer. This document discusses the role that Tissue factor plays in these processes and also the highly significant part it plays In angiogenesis, cancer, metastasis, inflammation, drug resistance, fertility and wound healing. Refer to Fig. 1.

### Forms of Tissue Factor

Two forms have been described: free TF (which includes a soluble, alternatively spliced and bloodborne TF) and membrane-bound TF. Soluble TF results from proteolytic cleavage at or near the linkage between the transmembrane and the extracellular domains of the TF molecule, forming protein fragments, whereas the other variants of free TF results from alternative splicing of the primary RNA transcript (Bogdanov et al.,2003; Bogdanov et al.,2006; Guo et al., 2001). These free forms of TF circulate In the plasma and are biologically active. Plasma TF can also be found circulating in association with cell-derived membrane microparticles, TF-bearing microparticles, which arise mainly from monocyte-macrophage membrane-lipid rafts or from regions of high raft content; In this case TF is called bloodbome. The circulating TF microparticles alone do not seem to effectively initiate coagulation, but when they bind and fuse with activated platelets via the mechanism Involving P-selectin glycoprotein ligand-1 on the microparticles and P-selectin on the platelets, they Initiate coagulation (del Conde, Shrimpton, Thiagarajan & Lopez, 2005).

The membrane-bound forms of TF Include both cellular TF (such as that found on monocytes, macrophages, endothelial and tumor cells) and lipid-vesicle-bound TF In urine [microparticles, exosomes] or in semen [prostasomes]. Cellular TF is found In three pools: surface, encrypted and intracellular On the plasma membrane of cells, TF resides mostly in a cryptic configuration; its release, which is often referred to as de-encryption, coincides with an increase in cell-surface phosphatidylserine, resulting In apoptosis and necrosis.

### Expression of TF

TF Is a constituent of both the subendothelial layer of the vascular wall and the extravascular tissue. It thereby forms a protective lining around the blood vessels and is ready to activate blood coagulation if vascular integrity is compromised (Ryan et al.,1992). Endothelial calls and blood monocytes (in contact with the bloodstream) do not constitutively express TF and do not have stores of TF.

Although TF is not normally expressed by cells within the bloodstream, gene transcription and subsequent protein expression can be Induced in monocytes, macrophages and endothelial cells by thrombin, endogenous inflammatory mediators such as interleukins 1 & 6, tumor-necrosis factor - alpha (TNF-α), vascular permeability factor (VPF), complement C5a, phorbol esters, plasma lipoproteins, plasma protein, collagen, immune complexes and microorganisms as well as other physiological and pathological mediators (Amirkhosravl et al., 1996; Østerud, Olsen & Wilsgard, 1990; Roth, 1994).

Platelets and lymphocytes can induce *de novo* TF biosynthesis in monocytes and endothelial cells. Likewise monocytes and natural killer cells can upregulate TF expression In endothelial cells (napoleone, Di Santo & Lorenzet,1997). This cell-cell cooperation is essential for TF induction in inflammatory disease and In the cell mediated immune response. Shearing forces and hypoxia also induce TF expression in many cells and provoke coagulation. TF activity also relies on changes in cellular phospholipid composition).

### Urinary Tissue Factor - a historical perspective

Urine is known to possess powerful 'procoagulant activity' [PCA] (Grunke et al, 1835) that normalizes the dotting time of haemophiliac patients *in vivo* (von Kaula et al, 1954). von Kaulla noted that preparations obtained by adsorbing human urine with barium sulphate were largely associated with materials that normalized the clotting of haemophiliac plasma *in vitro* (von Kaula et al,1965). It was demonstrated that minute amounts of these preparations had the ability to correct the dotting of patients who have a high titre of circulating 'antithromboplastin', now known as tissue factor pathway inhibitor [TFPI] (von Kaula et al, 1963). The PCA was first thought to be a tissue thromboplastin-related substance. It was later found that urinary PCA catalysed the conversion of prothrombin to thrombin only in the presence of platelet factor 3, factor V and Ca²⁺-a prothrombinase type of activity, and was therefore classified as a 'platelet cofactor' rather than TF (Matsumura et al, 1970, Caldwell et al,1963). Aoki and von Kaulla further investigated the interaction between platelet factor 3, factor V, prothrombin, and urinary PCA. They concluded that platelet factor 3 could be replaced by phospholipids in the activation of prothrombin with partially purified urinary procoagulant (uPC), provided that the lipid was used under optimal conditions. A similar observation was made by Joist and Alklaersig, who noted that phospholipid or platelets merely enhanced urinary PCA in the coagulation of plasma (Joist et al, 1967). Subsequently, a factor that had the properties of TF was described in urine. Kurosawa *et al.* purified the uPC by chromatography on phenyl-sepharose and showed that it promoted dot formation in a factor VII-dependent manner (Kurosawa et al, 1984). Aoki and von Kaulla suggested that the urinary PCA existed as small particles and they reported a factor with the properties of 'prothrombinase'. Subsequently, it was demonstrated that urinary PCA existed in lipid-associated vesicles and was mainly factor VII-dependent (Wiggins et al, 1987). The uPC can activate factor X in the presence of factor VII and calcium and was inhibited by concanavalin A (Zacharaski et al, 1974). Activity was restored by the addition of *á*-methyl-glucoside (Kurosawa et al, 1984). Later, it was confirmed that the procoagulant was TF by demonstrating almost total inhibition of the activity In the presence of a specific antibody to human TF (Carty et al, 1990). The activity appeared to be associated with membrane vesicles that passed through a 220 nM filter, but not one with 100 nM pores.

Following purification and the removal of contaminant Tamm-Horsfall mucoprotein, the procoagulant was found to exist in micro-aggregates. These micro-aggregates were composed of basic functional units of 151 kD consisting of two sub-units of 68 and 76 kD held together by disulphide bonds (Kurosawa et al, 1984). This contrasts with an established molecular weight for human TF of 43 kD. These differences probably reflect the relative impurity of the former preparation, since the mature TF mRNA transcript is the same In a variety of human tissues, including kidney. Indeed, a subsequent estimate of uTF using western blotting revealed that uTF has a similar molecular weight to TF derived from other human tissues [43 kD](Carty et al, 1990). The vesicles can be sedimented by ultracentrifugation (Carty 1990) and TF activity can be lecovered by solubilizing the TF-containing pellet in the detergent *â*-octyl-glucopyranoside (Lwaleed et al, 1999). The vesicles seemed to be intimately associated with fibrin strands when the latter were generated *in vitro* (Carty 1990) and showed specific binding to gold-labelled murine anti-TF (Lwaleed et al 1998). The labelling of these vesicles with gold-labelled annexin V (a placental anti-coagulant protein-I) showed that they contained not only TF, but also anionic phospholipids (Lwaleed et al, 1998), which could be the lipid provider for uTF activity. Indeed, uTF activity was inhibited In a dose-dependent manner when it was incubated with different concentrations of recombinant annexin V. This result agrees with those obtained for monocyte TF activity (Satta et al, 1994 and Carvalho et al, 1995).

### Sources of urinary TF

The predse source and the underlying biological mechanisms of uTF elevation in disease states remain poorly understood. Several studies have attempted to focus on this issue with different results. The demonstration that renal pelvis urine has normal procoagulant content excluded the lower urinary tract as a possible source (Matsumura et al, 1968). Two other possible sources of the uPC are implicated; uPC could be a tissue (excretory) product which passes through the glomerular filter, or be a secretory product of the kidney which originates in renal parenchymal tissue. uTF has a molecular weight of 43 kD and only a weak correlation has been observed between uTF levels and urinary protein excretion and markers of glomerular filtration rate (Lwaleed et al, 1998). Thus, uTF may not be blood-derived. In addition, uTF activity is not affected in clinical states such as haemophilia, heparin treatment and patients with circulating antithromboplastin. The kidney therefore remains a possible source of uPC. Several histochemical and immunolocalization studies have addressed the issue of the renal cell of origin of uTF. Some groups reported localization to the glomerulus (Bukovsky et al, 1992), while others reported signal within renal tubules (Lwaleed et al, 1998). The argument that the procoagulant is produced by renal tubules is supported by clinical observation and experimental models (Matsumura and von Kaulla, 1968; Matsumura et al, 1968; Matsuda et al, 1979; Lwaleed et al, 2007). We found, using a murine monoclonal antibody to human TF, that tubular cells showed a positive signal (Lwaleed et al, 1999). Tubular cells were also positive for annexin V (Lwaleed et al, 1999). It is also possible that renal macrophages and/or tubular cells become activated and secrete an increased amount of TF, particularly in disease states such as malignancy and certain types of inflammatory conditions (Carty et al, 1990). Since uTF elevation is similar in magnitude to that described for monocyte TF, It is reasonable to suggest that uTF levels may reflect the state of monocyte/macrophage 'activation' in disease states. A moderate association has been demonstrated between uTF and monocyte TF activity, especially with that obtained with lipopolysaccharide-stimulated cells (Lwaleed et al, 1998), but the stimulus for, and control of, uTf production remains to be established.

### uTF and Cancer

Simple tests for biomarker substances in the urine of patients with cancer are an attractive proposition (Wajsman et al (1975). uTF may be such a biomarker in certain circumstances (Carty, 1990, Adamson 1992 & 1993, Lwaleed 1996 & 1997, Adamson 1992, Collud, 1991 are examples). Although the mechanisms of uTF elevation In cancer patients are poorly understood, its level may be of clinical importance. Carty *et al*. reported increased uTF activity in patients with colorectal cancer, inflammatory bowel disease, and breast cancer. Although patients with benign colorectal and breast disease also had increase levels compared with normal controls or patients with rheumatoid arthritis, these were less than those in the malignant group. In patients with an abnormal colonoscopy 88 per cent had abnormal uTF levels, compared with 24 per cent of patients with normal colonoscopy. Adamson *et al*. showed higher levels of uTF in patients with malignant disease of the bladder and prostate than in controls and patients with benign prostatic hypertrophy. Lwaleed *et al.* showed no significant difference between normals, surgical controls or patients with benign (non-inflammatory) conditions. However, the controls showed significantly lower uTF levels than those with malignant or benign (inflammatory) conditions. Spillert and Lazaro, Østerud *et al.*, and Edwards *et al.* suggested that inflammatory conditions interfere with the host immune response, which leads to an Increase in monocyte TF expression, a mechanism which could similarly explain uTF elevation in these conditions. However, Carty *et al.* found that subjects with rheumatoid arthritis, all of whom had active disease, showed generally normal uTF levels. This suggests that uTF does not behave simply as an acute phase reactant.

uTF activity has shown some correlation to histological tumour grading and/or staging (breast, bladder, prostate, and colorectal cancer), bone scan status (prostate cancer), and serum prostate-specific antigen levels (prostate cancer) (Lwaleed, 1997). Patients with recurrent bladder malignancy also showed higher uTF levels than those with a normal check cystoscopy and levels were higher in patients who subsequently died (Adamson, 1992, Lwaleed, 1998).

### Structure of uTF

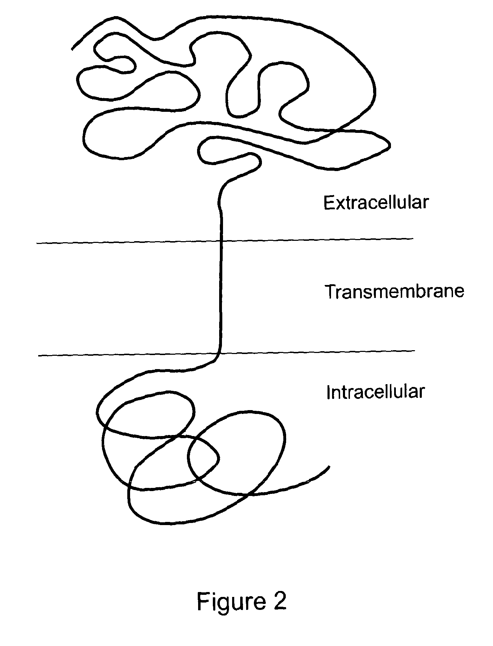

All full length membrane-bound tissue factors including uTF are single chain glycoproteins comprising 263 amino add residues with molecular weight of 43 to 45kD. They have three domains: 1) an extracellular domain; 2) a transmembrane domain; and 3) a C-terminal cytoplasmic intracellular domain (see Figure 2).

Each domain provides a critical function essential for the biology of uTF. The extracellular domain (aa 33-251) is responsible for binding to various proteins in the coagulation system such as Factor VII, TFPI and Factor X. The transmembrane domain (aa 252-274) is highly hydrophobic and anchors TF In the plasma membrane of cells. The intracellular domain (aa275-295) mediates signal transduction activity which leads to the activation and inactivation of various genes. The signal transduction function of tissue is vitally important in carcinogenesis, The expression of tissue in cancer cells is known to mediate the expression of VEGF and integrins. These mediators are important for angiogenesis, cell motility and metastasis. TF plays a role in tumour biology, including growth and spread. Activation of TF in malignant and certain inflammatory conditions may be reflected in its levels in a variety of body fluids. uTF levels are not significantly affected by age, sex or cigarette smoking (Lwaleed et al, 1999) which means that the number of false positive results will be low when screening large populations. Thus, uTF may be potentially useful in monitoring patients with renal disease and cancer.

### Methods for measuring urinary TF

### Activity Assays.

Advances in the understanding of the molecular structure and the biochemistry of TF have led to the development of assays for measuring various structural components of TF. Different methods have been used to assess TF at the cellular and plasmatic levels, and in other body fluids, with substantial clinical potential. A number of studies have employed assays that rely on the measurement of the procoagulant activity of uTF. These assays also only measure fully functional uTF and focus on the extracellular domain of uTF. One-stage and two-stage kinetic chromogenic assays (KCA) have been used to measure uTF procoagulant activity In disease states (Carty et al, 1990; Lwaleed et al, 1999), glomerulonephritis (Lwaleed et al, 1997a) and solid tumours (Lwaleed et al, 1997b). The effect of renal function on uTF measurements was studied by Lwaleed et al. (1998).

Changes in uTF activity have been demonstrated in several disease states. The uTF PCA, on the other hand, was slightly raised in patients with hyperthyroidism and was significantly increased in patients with Glomerulonephritis and cancer (Carty, 1990; Adamson 1992 &.1993; Colluci, 1991; Lwaleed 1996 & 1997, are examples). The uTF procoagulant activity was normal in thromboembolic and liver disease and markedly decreased In patients with parenchymal renal diseases and diabetes mellitus (QIH et al, 1996).

The KCA assays are manual assays more suitable for research applications but are less suitable for large scale clinical use and for instrument-based applications. Assays that measure uTF "activity" may not reflect the total amount of uTF present In a sample because some of the uTF may be structurally Inactive or proteolytically cleaved. These forms will not be measured in functional assays. Functional assays also preferentially measure the extracellular domain.

### Immunoassays.

An immunoassay is more suitable to clinical laboratories and a uTF-specific immunoassay would be most preferable for population screening. TF has been measured in urine and other body fluids using immunoassays such as ELISA formats (Fareed et al, 1995). Immunoassays utilize antibodies against the tissue factor protein to measure TF antigen or protein levels rather than the biological activity of TF. Most TF ELISAs in the past have used antibodies directed against the extracellular domain (e.g. American Diagnostica Inc's Imubind Tissue Factor ELISA (REF 845). The extracellular domain Is mainly responsible for the procoagulant activity of TF. Such ELISAs can measure various forms ofTF including full length TF, "soluble TF" which is formed by the proteolytic cleavage of the extracellular domain of TF and various other fragments that are produced by further proteolysis of TF. The presence of proteolytic fragments of TF in a sample would interfere with the measurement of full length TF (i.e. uTF) because the proteolytic fragments would block the binding of antibodies to the full length antibody. Other forms ofTF (i.e. alternately spliced TFs) which also have a native extracellular domain would also Interfere with the measurement of uTF for similar reasons.

Thus, to accurately measure uTF antigen In the presence of different forms ofTF and proteolytic fragments requires a different approach to the immunoassay. More specifically it requires a novel set of antibodies that will distinguish the uTF from the other TFs.

### Novel Approach for measuring uTF

uTF like all proteins is synthesized in the cell from N-terminal to C-terminal. Therefore, the presence of the C-terminal end of the uTF indicates that the whole molecule from N-terminal c-terminal has been synthesized. Thus, the C-terminal region of uTF, whether present intact with the whole uTF molecule or as a proteolytic fragment is a good indicator that the whole uTF molecule was synthesize. The c-terminal would also represent only the native uTF molecule and not the alternately spliced variants. Measuring the c-terminal region of uTF should *specifically* reflect the concentration of the whole uTF molecule and not the spliced variants.

We propose that the measurement of the c-terminal fragment of uTF would be a novel approach for accurately and specifically measuring the concentration of uTF in urine and other biological fluids. The measurement of the c-terminal region of uTF can be accomplished using various quantitative and qualitative immunoassay technologies including ELISA, latex agglutination assays, lateral flow technologies, etc. The important common feature of these assays are the development of polyclonal and monoclonal antibodies, antibody fragments, genetically engineered antibodies and/or other binding proteins that specifically target the c-terminal peptide. We propose that a specific immunoassay/assay using antibodies and specific binding molecules against the c-terminal peptide of uTF would be useful for the specific and accurate quantitation of uTF in biological fluids.

Carson and Yoder (Blood Coagul Fibrinolysis (1992) 3: 779-787) teach that monoclonal antibodies can be developed against the c-terminal fragment of TF. The utilized the monoclonal antibodies to show that the c-terminal peptide can be proteolytically cleaved from TF. They also used the antibodies to characterize the distribution of vesicles containing TF and the orientation of TF within the vesicles. They do not teach that c-terminal antibodies can be used for the quantitative or semi-quantitative measurement of the levels of TF in biological fluids. They also do not teach how to create antibodies to c-terminal TF peptide for use in a two site ELISA or other immunoassay format that can be used to develop an Immunoassay for measuring TF.

We propose the following methods for creating anti-c-terminal antibodies that can be used for developing two site ELISA for measuring uTF.

### Monoclonal Antibodies.

The c-terminal peptide of uTF is the 20 amino acid portion of the uTF protein that is below the plasma membrane and sticks out of the plasma membrane into the cytoplasmic portion of the cell. The key role of the c-terminal peptide is to meditate signal transduction as discussed above. We propose that two fragments of the c-terminal region (for example aa 275 - 285 and aa 286-295) be synthesized by standard methods. Polyclonal and monoclonal antibodies to each of the peptides are made using standard methodologies. Two-site immunoassays are developed using pairs of the antibodies to the peptides. We require that the antibodies to each of the peptides does not block each other when the intact uTF molecule Is present in the biological fluid or if the c-terminal 20 amino fragment is present without the rest of the uTF molecule.

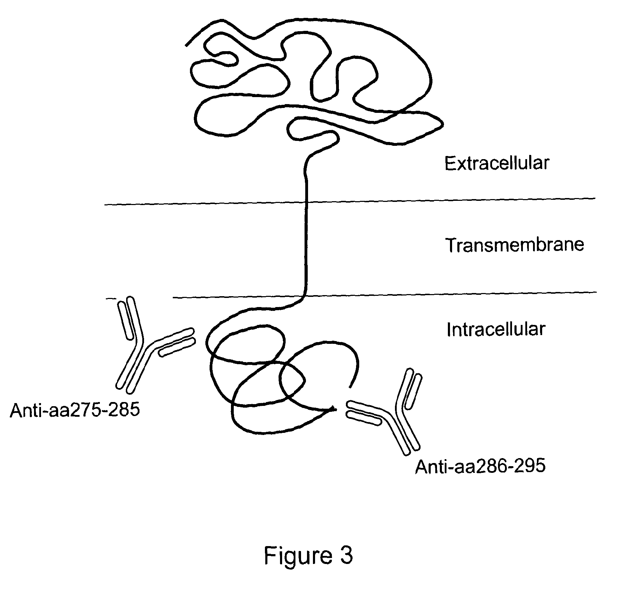

The anti-275-285 antibody can be used as the capture antibody for the ELISA and the anti-286-295 as the detection antibody for the ELISA. Refer to Fig.3.

### Sample Preparation

### Solubilization.

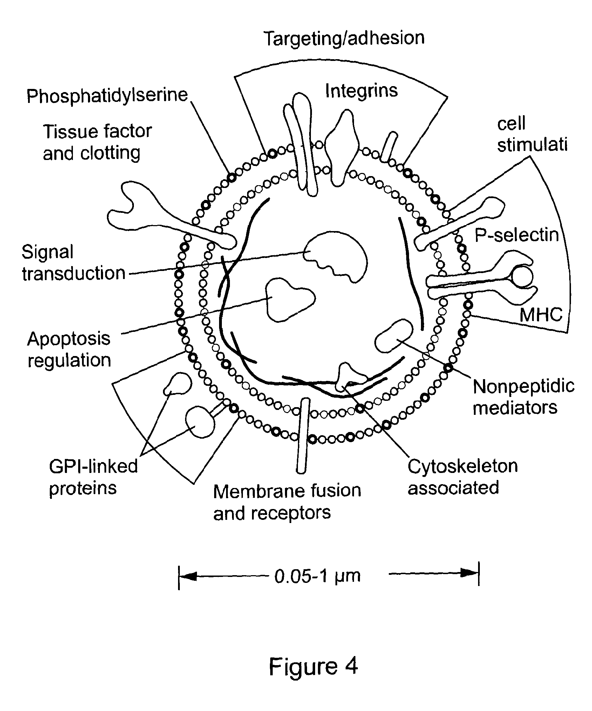

The uTF sits within a liposomal-like microparticle as depicted in Figure 4. In such a structure the c-terminal portion of the uTF molecule is inside the vesicle (Lwaleed et al 1992). It will be difficult for an antibody to bind to the c-terminal portion of uTF if it is within the vesicle. For measuring the c-terminal fragment of uTF in our proposed assay we propose to incorporate a step whereby the microvesicle or liposomal-like membranes which the uTF molecule sits in is solubilized by the addition of a detergent such as Triton X-100, NP-40, deoxycholate or tween 20. Solubilization of the microparticle by detergent will dissolve the microparticle structure and reveal the c-terminal fragment so that the antibodies can bind to the c-terminal fragment.

### Centrifugation.

Another sample preparation technique that can be used Is to concentrate the uTF microparticles before the immunoassay is performed. Micropartides are able to be separated from solution by high speed centrifugation. Typical g-forces to accomplish this is 10-20,000xg which can be performed in a standard laboratory microcentrifuge. Thus, it is possible to concentrate the microparticles by taking a urine sample of for example 1 ml and centrifuging at 12,000 x g to remove the microparticles from the urine solution. The pelleted microparticles containing uTF can then be solubilized in a small volume of detergent containing buffer (i.e. 50 µl). This procedure will concentrate the particles by 20-fold than what is present in urine.

### Preliminary Proof of Principle Studies.

We tried to use a commercial EUSA for tissue factor by American Diagnostica (ref 845) to measure tissue factor levels in urine. This ELiSA uses antibodies against the extracellular portion of TF for both capture and detection. We were not able to measure TF in urine using this commercial assay. These findings suggest that a different combination of antibodies are needed to detect TF in urine.

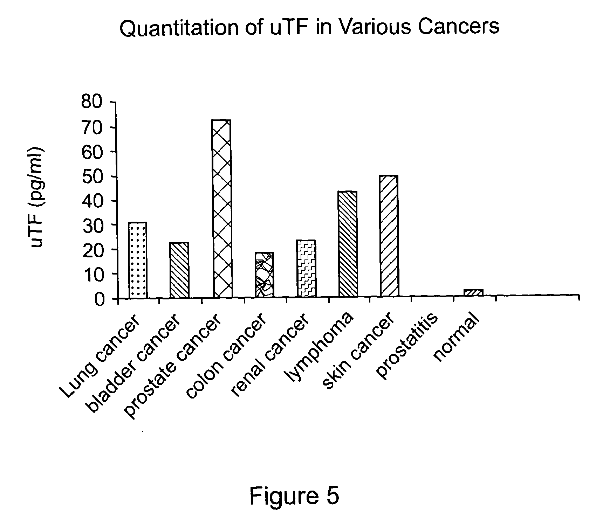

We have performed preliminary studies using ELISA technology to demonstrate the proof of principle that antibodies to the c-terminal peptide of uTF can be useful in quantitation of uTF in urine of cancer patients. In this study we used a polyclonal antibody to c-terminal fragment of tissue factor as the capture antibody In an ELISA. The detection antibody in this study was a monoclonal antibody against the extracellular domain. We believe that the ELISA assay is more sensitive in determining differences between malignant and Inflammatory conditions. Urines were obtained from commercial sources from patients with previously diagnosed cancers of various types. Urine samples 1.5 ml was centrifuged In microcentrifuge and the pelleted microparticles were re-suspended in a detergent containing buffer. The dissolved microparticle solution was run in the ELISA. Fig. 5 shows that higher concentrations of c-terminal peptide contain uTF was present in urines from lung, bladder, prostate colon, renal lymphoma and skin cancers. Normal (non-cancerous) patients and patients with prostatitis (an inflammatory condition of the prostate) had low levels of uTF.

### Potential methods to optimize quantitation of uTF

The quantitation of uTF may still be optimized by a more sensitive ELISA. This may be accomplished by using a different pair of antibodies than in Figure 5. A better ElI5A may be constructed using two high affinity monoclonal and/or polyclonal antibodies with high specificity directed against different regions of the c-terminal fragment of uTF.

Another improvement in the measuring of uTF may be gained by using a "normalization" factor in urine. For example, it may be useful to normalize the quantity of uTF using the amount of protein in the urine sample. The protein in the sample can be measured using standard assays (BCA, coomassie blue reagent). The amount of uTF determined by ELISA divided by the amount of protein may provide a better metric to discriminate urines derived from patients with or without cancer. Another possible method to "normalize' the uTF amount is to determine the ration of uTF activity divided by the uTF protein content. The ratio of activity/protein may provide a better discriminator of patient with cancer vs non-cancer conditions. Activity for uTF can be measured by any of the methods described above.

### Summary

We provide a novel technological approach for quantitation of c-terminal fragment of uTF for the purpose of quantitation, diagnosis and population screening of cancer. We provide a rationale for measuring c-terminal fragment, methods for making assays, monoclonal and polyclonal antibodies, together with accepted methods of sample preparation. Taken together these proposals and ideas constitute a novel approach for diagnosis and population screening for cancers using urine from cancer patients. In addition we believe that our approach may be useful in the diagnosis of other pathological conditions.

### List of Figures

*Figure 1* *Cellular tissue factor initiated coagulation*
   *Factor VII binds to TF on TF-bearing cells and is auto-activated to VIIa. The resulting complex activates Factor X and Factor IX and a small amount of Thrombin IIa. A feedback mechanism (TFPI) inhibits the TF: VIIa complex. However the small amount of thrombin generated activates platelets and Factor V, releases and activates Factor VIII from being bound to von Willebrand factor and activates Factor XI. Activated Factor IX binds to the activated platelets and activates Factor X. Platelet generated Factor X then generates large amounts of Thrombin which is needed for the clot to form.*
*Figure 2* -A model of TF showing a representation of the three domains
*Figure 3* *Showing two antibodies*
*Figure 4* *From:* Hugel et al. Physiology (2005) 20:22-27*.*
*Figure 5**. Quantitation of Urinary Tissue Factor by ELISA in various cancers and normal individuals.*

## Claims

1. A method for detecting the c -terminal region (aka intracellular domain) of urinary tissue factor (uTF) in a sample comprising: a) binding an anti-uTF antibody against the amino acid sequence 275-285 within the c -terminal region of human uTF to a solid phase; b) adding the sample to the solid phase, wherein uTF is present in the sample which binds to the antibody; c) adding an anti-uTF antibody against the amino acid sequence 286-295 within the c-terminal region human uTF; and d) detecting uTF by direct or indirect immunolabelling of the second anti-uTF antibody.

2. The method of claim 1, wherein detecting c-terminal region of uTF is by indirect immunolabelling of the c-terminal region anti-uTF antibody.

3. The method of claim 2, wherein the capture anti-uTF antibody is raised in a first species and wherein a labelled anti-uTF antibody raised in the same species as the first species is added to the solid phase and detected.

4. A method for measuring the amount of c-terminal region of uTF in a sample comprising the method of claim 2 and further comprising: e) quantifying anti-uTF antibody detected, thereby measuring the amount of c-terminal region of uTF in the sample.

5. A method for diagnosing cancer in a subject comprising a) measuring the amount of uTF in a test sample from the subject according to the method of claim 3; and b) comparing the amount of c-terminal region of uTF in the test sample to the amount of c-terminal region of uTF in a control sample having normal c-terminal region of c-terminal region of uTF; wherein cancer is diagnosed by an increased amount of c-terminal region of uTF in the test sample compared with the control sample.

6. The method of claim 1 or claim 5, wherein the test sample is selected from the group consisting of urine, saliva, whole blood, plasma, platelet rich plasma: (PRP), platelet poor plasma (PPP), pooled normal plasma (PNP).

7. The method of claim 1 or claim 5, wherein the solid phase is a membrane, plate, microwell or bead.

8. The method of claim 1 or claim 5, wherein detecting c-terminal region of uTF is by direct immunolabelling of the anti-uTF antibody.

9. The method of claim 6 or claim 8, wherein the c-terminal region anti-uTF antibody is labelled with horseradish peroxidase.

10. The method of claim 2 or claim 4, wherein the c-terminal region of anti-uTF antibody is raised in a first species and wherein a labelled antibody raised in a second species against antibodies from the first species is added to the solid phase and detected.

## Patentansprüche

1. Verfahren zum Erkennen der c-terminalen Region (auch bekannt als intrazelluläre Domäne) des Uringewebefaktors (uTF) in einer Untersuchungsrobe, folgendes umfassend: a) Binden eines anti-uTF Antikörpers gegen die Aminosäuresequenz 275-285 innerhalb der c-terminalen Region des menschlichen uTF an eine Festphase; b) Beigabe der Untersuchungsrobe zur Festphase, wobei uTF in der Untersuchungsrobe vorhanden ist, die sich an den Antikörper bindet; c) Beigabe eines anti-uTF Antikörpers gegen die Aminosäuresequenz 286-295 innerhalb der menschlichen uTF der c-terminalen Region; und d) Erkennen des uTF durch direkte oder indirekte Immunmarkierung des zweiten anti-uTF Antikörpers.

2. Verfahren nach Anspruch 1, wobei das Erkennen der c-terminalen Region des uTF durch indirekte Immunmarkierung des anti-uTF Antikörpers der c-terminalen Region erfolgt.

3. Verfahren nach Anspruch 2, wobei das Erfassen des anti-uTF Antikörpers in einer ersten Art gezüchtet wird, und wobei ein markierter anti-uTF Antikörper, der in derselben Art gezüchtet wird, wie die erste Art, der Festphase beigegeben und erkannt wird.

4. Verfahren zum Messen des Umfangs der c-terminalen Region des uTF in einer Untersuchungsprobe, das Verfahren nach Anspruch 2 umfassend, und darüber hinaus folgendes umfassend: e) Quantifizieren der erkannten anti-uTF Antikörper, und damit Messen des Umfangs der c-terminalen Region des uTF in der Untersuchungsprobe.

5. Verfahren zum Diagnostizieren von Krebs bei einem Patienten, folgendes umfassend a) Messen des Umfangs von uTF in einer Untersuchungsprobe des Patienten gemäß dem Verfahren nach Anspruch 3; und b) Vergleichen des Umfangs der c-terminalen Region von uTF in der Untersuchungsprobe mit dem Umfang der c-terminalen Region von uTF in einer Kontrollprobe, die eine normale c-terminale Region der c-terminalen Region von uTF aufweist; wobei Krebs durch einen erhöhten Umfang der c-terminalen Region von uTF in der Untersuchungsprobe im Vergleich zur Kontrollprobe diagnostiziert wird.

6. Verfahren nach Anspruch 1 oder Anspruch 5, wobei die Untersuchungsprobe aus einer Gruppe ausgewählt wird, die sich aus Urin, Speichel, Vollblut, Plasma, plättchenreichem Plasma: (PRP), plättchenarmem Plasma (PPP), normalem Poolplasma (PNP) zusammensetzt.

7. Verfahren nach Anspruch 1 oder Anspruch 5, wobei die Festphase eine Membran, Platte, Mikrotiter oder Wulst ist.

8. Verfahren nach Anspruch 1 oder Anspruch 5, wobei das Erkennen der c-terminalen Region von uTF durch direkte Immunmarkierung des anti-uTF Antikörpers erfolgt.

9. Verfahren nach Anspruch 6 oder Anspruch 8, wobei der anti-uTF Antikörper der c-terminalen Region mit Meerrettich-Peroxidase markiert wird.

10. Verfahren nach Anspruch 2 oder Anspruch 4, wobei die c-terminale Region des anti-uTF Antikörpers in einer ersten Art gezüchtet wird, und wobei ein markierter Antikörper, der in einer zweiten Art gegen Antikörper der ersten Art gezüchtet wurde, zur Festphase beigegeben und erkannt wird.

## Revendications

1. Procédé de détection de la région C-terminale (domaine intracellulaire aka) du facteur de tissu urinaire (uTF) dans un échantillon, comprenant a) la liaison d'un anticorps anti-uTf contre la séquence d'acides aminés 275-285 dans la région C-terminale de l'uTF humain à une phase solide ; b) l'addition de l'échantillon à la phase solide, dans lequel l'uTF est présent dans l'échantillon qui se lie à l'anticorps ; c) l'addition d'un anticorps anti-uTF contre la séquence d'acides aminés 286-295 dans l'uTF humain de la région C-terminale ; et d) la détection de l'uTF par immunomarquage direct ou indirect du second anticorps anti-uTF.

2. Procédé selon la revendication 1, dans lequel la détection de la région C-terminale de l'uTF se fait par immunomarquage de l'anticorps anti-uTF de la région C-terminale.

3. Procédé selon la revendication 2, dans lequel l'anticorps anti-uTF de capture est développé dans une première espèce et dans lequel un anticorps anti-uTF marqué développé dans la même espèce que la première espèce est ajouté à la phase solide et détecté.

4. Procédé de mesure de la quantité de région C-terminale de l'uTF dans un échantillon, comprenant le procédé de la revendication 2 et comprenant en outre e) la quantification de l'anticorps anti-uTF détecté en mesurant de la sorte la quantité de région C-terminale de l'uTF dans l'échantillon.

5. Procédé de diagnostic du cancer chez un sujet, comprenant a) la mesure de la quantité d'uTF dans un échantillon d'essai provenant du sujet selon le procédé de la revendication 3 ; et b) la comparaison de la quantité de région C-terminale de l'uTF dans l'échantillon d'essai à la quantité de région C-terminale de l'uTF dans un échantillon témoin ayant une région C-terminale normale de la région C-terminale de l'uTF ; dans lequel le cancer est diagnostiqué par une quantité accrue de région C-terminale de l'uTF dans l'échantillon d'essai en comparaison de l'échantillon témoin.

6. Procédé selon la revendication 1 ou la revendication 5, dans lequel l'échantillon d'essai est choisi dans le groupe constitué de l'urine, de la salive, du sang entier, de plasma, du plasma riche en plaquettes (PRP), du plasma déplaquetté, (PPP), d'un pool de plasma normal (PNP).

7. Procédé selon la revendication 1 ou la revendication 5, dans lequel la phase solide est une membrane, une plaquette, un micro-puits ou une bille.

8. Procédé selon la revendication 1 ou la revendication 5, dans lequel la détection de la région C-terminale de l'uTF se fait par immunomarquage direct de l'anticorps anti-uTF.

9. Procédé selon la revendication 6 ou la revendication 8, dans lequel la région C-terminale de l'anticorps anti-uTF est marquée par de la peroxydase de raifort.

10. Procédé selon la revendication 2 ou la revendication 4, dans lequel la région C-terminale de l'anticorps anti-uTF est développée dans une première espèce et dans lequel un anticorps marqué développé dans une seconde espèce contre les anticorps provenant de la première espère est ajouté à la phase solide et détecté.
